# EUROPEAN PATENT APPLICATION

(11) **EP 4 461 737 A1**
(43) Date of publication of application: **13.11.2024**
(21) Application number: 22916735.8
(22) Date of filing: 28.12.2022
(51) Int. Cl.: C07K 7/06, C07K 7/08, C07K 14/00, A61K 47/42, A61P 35/00

(54) **POLYPEPTIDE FOR DELIVERING ANTIGEN TO IMMUNE CELLS**

(30) Priority: 28.12.2021 KR 20210189421
(71) Applicant: JW Shinyak Corporation, Gwacheon-si, Gyeonggi-do 13840 (KR)
(72) Inventor: JEON, Yoon Jae, Gwangju-si Gyeonggi-do 12781 (KR); KIM, Younghoon, Namyangju-si Gyeonggi-do 12186 (KR); HONG, Seungho, Suwon-si Gyeonggi-do 16380 (KR)
(74) Representative: Mewburn Ellis LLP
(86) International application number: PCT/KR2022/021480
(87) International publication number: WO 2023/128594

(57) **Abstract**

The present invention relates to a polypeptide for delivering an antigen to immune cells and, specifically, to: a novel polypeptide comprising a cell membrane penetrating peptide and a peptide binding to a surface molecule on immune cells; a fusion polypeptide in which an antigen is coupled to the polypeptide; a nucleic acid coding for the polypeptide or the fusion polypeptide; an immune cell sensitized with the fusion polypeptide or the nucleic acid coding therefor; and an immunotherapeutic agent, antitumor or anticancer vaccine, and a composition for treating a tumor or cancer, each comprising the immune cell.

## Description

### [Technical Field]

The present invention relates to a polypeptide for delivering an antigen to an immune cell, and specifically, a novel polypeptide comprising a cytoplasmic transduction peptide and a peptide binding to the surface molecule of the immune cell, a fusion polypeptide comprising the polypeptide bound with an antigen, a nucleic acid encoding the polypeptide or the fusion polypeptide, an immune cell sensitized by the fusion polypeptide or the nucleic acid encoding the same, and an immunotherapeutic agent, an anti-tumor or anti-cancer vaccine, and a composition for treating tumors or cancer, each containing the immune cell.

### [Background Art]

The signal sequence (membrane transduction sequence: MTS) or cytoplasmic penetration peptide (CPP) present in fibroblast growth factor (FGF) is known to have the property of penetrating cell membranes. The signal sequence has the property of easily penetrating the cell membranes, but does not enter the nucleus due to the lack of a nuclear localization sequence (NLS).

MTS or CPP may be used as an antigen carrier. Peptide antigens introduced into the cytoplasm may be processed through the proteasome and presented on the cell surface carried via MHC class I. In addition, MTS or CPP may be used to deliver therapeutic substances for delivering proteins with specific functions developed to target various signal transduction processes that regulate cell death and cell growth that occur in the cytoplasm.

In this regard, the inventors of the present application developed a cytoplasmic transduction peptide (CTP) that has an ability to transduce cell membranes and remains in the cytoplasm, and found that the CTP is suitable for inducing a cytotoxic T lymphocyte (CTL)-response and can be used as an effective drug delivery system (DDS) into the cytoplasm because it has excellent cell membrane-penetrating ability and remains in the cytoplasm (Korean Patent No. 0608558).

Meanwhile, *Staphylococcus aureus* is widely distributed in the natural world due to strong resistance thereof to the natural environment and is present in the purulent drainage of humans and animals as well as on the skin of healthy humans and animals, and thus has a very high risk of contaminating food and infecting humans. Pathogenic factors produced by *Staphylococcus aureus* produce exotoxins, enterotoxins, hemolysin toxins, leucocidins, and epidermolytic toxins, as well as extracellular products such as toxic shock syndrome toxin (TSST), thus causing various important diseases in humans, such as food poisoning.

Staphylococcal enterotoxin type B (also referred to "SEB") is a toxin produced by the *Staphylococcus aureus* toxin-type food poisoning bacteria, which is distributed worldwide and mainly causes food poisoning, abnormal immune responses and severe immune diseases.

SEB has a protein having a small molecular weight of about 26 to 29 kDa in size, but is highly soluble in water, has strong resistance to proteases such as pepsin, trypsin, and papain, and is a very stable toxin that withstands extreme temperatures or pH.

Regarding the functions, SEB, which is also referred to as superantigen (Sag), is an antigen that serves to link the T-cell receptor (TCR) present on activated CD4+ or CD8+ T cells to the major histocompatibility complex class II present in antigen-presenting cells (APCs). SEB binds to the MHC class II of macrophages and then also binds to the Vb8 region of the T cell receptor, thereby abnormally activating T cells, secreting great amounts of inflammatory cytokines such as IL-6, IL-1β, and MIP-1 (macrophage inflammatory protein-1), and inducing an abnormal immune response. It is known that, when the respiratory system is exposed to SEB, the number of people who die from SEB is small, but more than 80% of those exposed to SEB show clinical symptoms, and the symptoms are too severe to maintain normal daily life for 1 to 2 weeks.

Under this technical background, the present inventors selected peptides binding to MHC class II through structural research of cytoplasmic transduction peptides and SEB, and found that a novel polypeptide comprising a cytoplasmic transduction peptide and a peptide binding to surface molecules of immune cells can improve the yield of antigen delivery to immune cells. Based thereon, the present invention was completed.

### [Disclosure]

Therefore, the present invention has been made in view of the above problems, and it is one object of the present invention to provide a novel polypeptide for delivering antigens to immune cells.

It is another object of the present invention to provide a nucleic acid encoding the polypeptide.

### It is another object of the present invention to provide a fusion polypeptide in which an antigen is bound to the polypeptide.

It is another object of the present invention to provide a nucleic acid encoding the fusion polypeptide.

It is another object of the present invention to provide an immune cell containing the fusion polypeptide or the nucleic acid.

It is another object of the present invention to provide an immunotherapeutic agent containing the immune cell.

It is another object of the present invention to provide an anti-tumor or anti-cancer vaccine containing the immune cell.

It is another object of the present invention to provide a composition for treating tumors or cancer containing the immune cell. It is another object of the present invention to provide a method for treating tumors or cancer comprising administering the immune cell to a subject. It is another object of the present invention to provide the use of the immune cell for the preparation of a composition for treating tumors or cancer.

In accordance with one aspect of the present invention, the above and other objects can be accomplished by the provision of a polypeptide for delivering an antigen to an immune cell comprising a cytoplasmic transduction peptide (CTP) and a peptide binding to a surface molecule of the immune cell.

In accordance with another aspect of the present invention, provided is a nucleic acid encoding the polypeptide.

In accordance with another aspect of the present invention, provided is a fusion polypeptide in which an antigen is bound to the polypeptide.

In accordance with another aspect of the present invention, provided is a nucleic acid encoding the fusion polypeptide.

In accordance with another aspect of the present invention, provided is an immune cell containing the fusion polypeptide or the nucleic acid encoding a fusion polypeptide.

In accordance with another aspect of the present invention, provided is an immunotherapeutic agent containing the immune cell.

In accordance with another aspect of the present invention, provided is an anti-tumor or anti-cancer vaccine containing the immune cell.

In accordance with another aspect of the present invention, provided is a composition for treating tumors or cancer containing the immune cell. In accordance with another aspect of the present invention, provided is a method for treating tumors or cancer comprising administering the immune cell to a subject. In accordance with another aspect of the present invention, provided is the use of the immune cell for the preparation of a composition for treating tumors or cancer.

### [Description of Drawings]

FIG. 1 shows the result of comparison in MoDC permeability between CTP/CTP-SEB groups.
FIG. 2 shows the difference in MoDC permeability between CTP/CTP-SEB groups.
FIG. 3 shows a vector for producing recombinant fusion proteins.
FIG. 4 shows the results of purification and the results of QC analysis using Ni-NTA resin in the production of recombinant fusion proteins.
FIG. 5 shows the results of measurement of cell permeability of the fusion protein.
FIG. 6 shows the results of analysis of direct binding force of the fusion protein.
FIG. 7 shows the results of analysis of the amount of protein remaining in the culture medium.
FIG. 8 shows DC phenotypes and cell membrane permeability comparison of PTD-based peptides.
FIG. 9 shows the results of antigen-specific T cell proliferation analysis.
FIG. 10 shows the results of determination of the antitumor effects in mice inoculated with dendritic cells sensitized with CTP-OVA or SCTP-OVA antigen.
FIG. 11 shows the results of analysis of permeability depending on the type of immune cells.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

In one aspect, the present invention is directed to a polypeptide for delivering an antigen to an immune cell comprising a cytoplasmic transduction peptide (CTP) and a peptide binding to a surface molecule of the immune cell.

The cytoplasmic transduction peptide may, for example, comprise at least one selected from protein transport domains such as CTP (cytoplasmic transduction peptide, cytoplasmic-residual cytoplasmic transduction peptide), HP4, Hph-1, Mph-1, Sim-2, Tat, VP22, Antp (Antennapedia), Pep-1 (peptide), PTD-5, R9 (arginine), and 7R domains, but is not limited thereto.

Specifically, the cytoplasmic transduction peptide may be a cytoplasmic-residual cytoplasmic transduction peptide that has the ability to penetrate the cell membrane, retains the ability to penetrate the cell membrane even when the cells treated with the cytoplasmic transduction peptide are treated with proteases after a predetermined period of time, and remains in the cytoplasm after penetrating the cell membrane. When the cytoplasmic transduction peptide is fused with a peptide binding to a molecule on the surface of an immune cell, if endocytosis and ubiquitin are inhibited, the polypeptide is not degraded and exists within the cells.

The cytoplasmic transduction peptide may have a length of, for example, 9-20 aa, more preferably 9-15 aa, and most preferably about 11 aa.

For example, the cytoplasmic transduction peptide may comprise a sequence selected from the group consisting of SEQ ID NOS: 5 to 7. The present inventors found that, when a cytoplasmic transduction peptide comprising a sequence selected from the group consisting of SEQ ID NOS: 5 to 7 is fused with a peptide that binds to the surface molecule of an immune cell, it exhibits superior cell membrane-penetrating ability compared to the cytoplasmic transduction peptide.

| Type of peptide | Sequence | No. |
|---|---|---|
| CTP512 | YGRRARRRRRR | 5 |
| CTP509 | YKRKARRAARR | 6 |
| CTP513 | YGRRRRRRRRR | 7 |

For example, the peptide binding to the surface molecule of the immune cells may be a peptide binding to a major histocompatibility complex (MHC) molecule. Specifically, the peptide binding to the surface molecule of the immune cells may comprise a peptide binding to MHC (major histocompatibility complex) class II (MHC class II) expressed as a surface molecule of the immune cells.

In one embodiment, the peptide binding to the surface molecule of the immune cells may comprise a sequence selected from the group consisting of SEQ ID NOS: 1 to 4.

| Type of peptide | Sequence | No. |
|---|---|---|
| SEB1 | KSIDQFLYFDLY | 1 |
| SEB2 | RYSIKDTKLGN | 2 |
| SEB3 | YYQCYFSKKTNDINSHQTDKRKTCM | 3 |
| SEB4 | YNKKKATVQELD | 4 |

The antigen may be a cancer or tumor antigen for activating an immune response against cancer or tumor. The antigen is a cancer or tumor antigen that may induce cancer- or tumor-specific immune cells, such as cytotoxic T cells, in cell therapy. The cancer or tumor antigen may be, for example, a self or non-self-antigen. For example, cancer or tumor self-antigens derived from the genes of patients may comprise hTERT (GenBank: BAC11010.1), WT1 (GenBank: AAO61088.1), NY-ESO1 (GenBank: CAA05908.1), MAGE-A3 (NCBI Reference Sequence: NP_005353.1), cancer-specific mutant antigens comprise tumor-suppressing or inducing genes such as neoantigens, mutated P53 and RAS, and exogenous cancer or tumor antigens may comprise cancer- or tumor-causing viral antigens such as CMV, EBV and HPV.

hTERT, a self-cancer or tumor antigen, is an enzyme that synthesizes telomeric DNA at the ends of chromosomes, which cancer cells excessively active to evade telomere-dependent cell death, and which is known as a target antigen for various solid cancers comprising lung, stomach and pancreatic cancer (Kim NW, et al. Science. 1994;266:2011-2015), and WT1 is a gene related to Wilms tumor that encodes a zinc finger transcription factor, which is a protein involved in cell proliferation and differentiation, apoptosis, and organ development, and is known as a target antigen for cerebrospinal cancer, lung cancer, or the like (Call KM, et al., Cell. 1990. 60:509-520; Nakahara Y, et al., Brain Tumor Pathol. 2004. 21:113-6). In addition, NY-ESO1 is a protein belonging to CTA (cancer testis antigen), which is well known to be expressed mainly in various cancer cells, comprising germ cells, sarcoma, and breast cancer (Gnjatic S, et al., Adv Cancer Res. 2006;95:1-30). MAGE-A3 is a protein belonging to the melanoma-associated antigen family, which is known to be overexpressed in various cancer cells comprising lung cancer, sarcoma, and melanoma, and is considered as a suitable target antigen for cancer immunotherapy (Decoster L, et al., Ann Oncol. 2012 Jun;23(6):1387-93).

In some cases, the antigen may be, for example, A33, ALK, alpha-fetoprotein (AFP), adrenergic receptor beta 3 (ADRB3), alpha-folate receptor, AD034, AKT1, BCMA, beta-human chorionic gonadotropin, B7H3(CD276), BST2, BRAP, CD5, CD13, CD19, CD20, CD22, CD24, CD30, CD33, CD38, CD40, CD44v6, CD52, CD72, CD79a, CD79b, CD89, CD97, CD123, CD138, CD160, CD171, CD179a, carbonic anhydrase IX (CAIX), CA-125, carcinoembryonic antigen (CEA), CCR4, C-type lectin-like molecule-1 (CLL-1 or CLECL1), claudin 6 (CLDN6), CXORF61, CAGE, CDX2, CLP, CT-7, CT8/HOM-TES-85, cTAGE-1, ERBB2, epidermal growth factor receptor (EGFR), EGFR variant III (EGFRvIII), epithelial cell adhesion molecule (EPCAM), elongation factor 2 mutated (ELF2M), ephrin type-A receptor 2 (EphA2), EMR2, Fms-like tyrosine kinase 3 (FLT3), FCRL5, Fibulin-1, G250, GD2, glycoprotein 36 (gp36), glycoprotein 100 (gp100), glucocorticoid-induced tumor necrosis factor receptor (GITR), GPRC5D, GloboH, G protein-coupled receptor 20 (GPR20), GPC3, hsp70-2, high molecular weight-melanoma-associated antigen (HMWMAA), hepatitis A virus cell receptor 1 (HAVCR1), HAGE, HCA587/MAGE-C2, hCAP-G, HCE661, HER2/neu, HLA-Cw, HOM-HD-21/Galectin9, HOM-MEEL-40/SSX2, HOM-RCC-3.1.3/CAXII, HOXA7, HOXB6, Hu, HUB 1, insulin growth factor (IGF 1)-IL, IGF-II, IGF receptors, interleukin-13 receptor subunit alpha-2 (IL-13Ra2 or CD213A2), interleukin 11 receptor alpha (IL-11Ra), IGLL1, KIT(CD117), KM-HN-3, KM-KN-1, KOC1, KOC2, KOC3, LAGA-1a, LAGE-1, LAIR1, LILRA2, LY75, Lewis Y antigen, MUC1, MN-CA IX, M-CSF, MAGE-1, MAGE-4a, mesothelin, MAGE-A1, MAD-CT-1, MAD-CT-2, MART1, MPPl 1, MSLN, neural cell adhesion molecule (NCAM), NY-ESO-1, NY-ESO-5, Nkp30, NKG2D, mammary differentiation antigen (NY-BR-1), NY-BR-62, NY-BR-85, NY-CO-37, NY-CO-38, NNP-1, NY-LU-12, NY-REN-10, NY-REN-19/LKB/STK1 1, NY-REN-21, NY-REN-26/BCR, NY-REN-3/NY-CO-38, NY-REN-33/SNC6, NY-REN-43, NY-REN-65, NY-REN-9, NY-SAR-35, o-acetyl-GD2 ganglioside (OAcGD2), OGFr, PSMA, prostatic acid phosphatase (PAP), p53, prostate-carcinoma tumor antigen-1 (PCTA-1), prostate stem cell antigen (PSCA), serine protease 21 (testisin or PRSS21), platelet-derived growth factor receptor beta (PDGFR-beta), PLAC1, pannexin 3(PANX3), PLU-1, ROR-1, RAGE-1, RU1, RU2, Rab38, RBPJkappa, RHAMM, stage-specific embryonic antigen-4 (SSEA-4), SCP1, SSX3, SSX4, SSX5, Tyrp-1, TAG72, thyroglobulin, 5T4, tumor-associated glycoprotein 72 (TAG72), tyrosinase, transglutaminase 5 (TGS5), TEM1, TEM7R, thyroid-stimulating hormone receptor (TSHR), Tie 2, TRP-2, TOP2A, TOP2B, uroplakin 2 (UPK2), vimentin, or vascular endothelial growth factor receptor 2 (VEGFR2).

In addition, the antigen is a pathogen (bacteria, virus, etc.), chemical compound, pollen, cancer cell, shrimp, or the like, or some peptide or protein thereof, more preferably a cancer antigen peptide, or any substance causing an immune response in the body, but is not limited thereto. The antigen may preferably be a protein, recombinant protein, glycoprotein, gene, peptide, polysaccharide, lipopolysaccharide, polynucleotide, cell, cell lysate, bacteria, virus, or the like, and more preferably, a cancer antigen peptide. The protein may be an antibody, antibody fragment, structural protein, regulatory protein, transcription factor, toxin protein, hormone, hormone analog, enzyme, enzyme fragment, transport protein, receptor, receptor fragment, biodefense inducer, storage protein, movement protein, exploitive protein, reporter protein or the like. Any substance may be used without limitation as long as it can act as an antigen in the living body and induce an immune response.

The antigen according to the present invention is an antigen that can bind to the polypeptide according to the present invention and comprises inactivated tumor cells, tumor cell-related genes produced by genetic recombination, peptides, or proteins. In order to obtain an antigen by genetic recombination, a known nucleotide sequence encoding the antigen, a full length of the known sequence or a portion of the full length may be used. The nucleotide sequence encoding the antigen may be cloned into a vector to express the desired antigen.

The antigen may bind to the N-terminus or C-terminus of the polypeptide. The covalent binding may be performed using methods known in the art depending on the type of antigen and may be obtained, for example, by cloning and intracellularly expressing genes encoding polypeptides.

In some cases, linkers that do not interfere with the activity of the polypeptide, for example, N-succinimidyl iodoacetate, N-maleimidobutyryl oxysuccinamide ester, 1,5-difluoro-2,4-dinitrobenzene, bis(diazo)benzidine, 3,3-dithio-bis-(sulfosuccinimidyl-propionate), ethylene glycol bis(sulfosuccinimidyl succinate), dicyclohexyl carbodiimide and the like may be used, but are not limited thereto. On the other hand, if the antigen is active only when decomposed from the polypeptide, a linker that can be cleaved *in vivo* is used. For example, binders with carboxylic acid ester and/or disulfide bonds may be used.

In one embodiment, the immune cells may be antigen-presenting cells (APC) that present on the surface thereof the antigen delivered by the polypeptide according to the present invention. For example, the immune cells may be dendritic cells, monocytes, Wrangel Hans cells, macrophages, T cells, or B cells, but are not limited thereto.

For example, the peptide that binds to the surface molecule of the immune cells may be fused to one end of the cytoplasmic transduction peptide. The peptide that binds to the surface molecule of the immune cells may be bound to the N-terminus or C-terminus of the cytoplasmic transduction peptide.

In some cases, the peptide that binds to the surface molecule of the immune cells may be bound to the cytoplasmic transduction peptide via a linker. The linker may be a peptide linker and may have a length of about 5-25 aa, specifically about 5-10 aa. For example, the linker may comprise hydrophilic amino acids such as glycine and/or serine, but is not limited thereto.

Specifically, in order to provide structural flexibility, the linker comprises, for example, a glycine linker (G, Gly)ₚ (in which p is 1 to 10) and a GS linker (GₙS)ₘ (in which n and m are each 1 to 10) . Specifically, the linker may comprise GGGGS or (GGGGS)₂, or may comprise a 5-10 aa glycine of (G, Gly)ₚ in which p is 5 to 10.

In another aspect, the present invention is directed to a vector comprising the nucleic acid.

The term "nucleic acid" is intended to encompass both DNA (gDNA and cDNA) and RNA molecules, and a nucleotide, which is the basic constituent unit of the nucleic acid, comprises naturally derived nucleotides as well as analogues thereof, in which sugar or base moieties are modified. The sequence of the nucleic acid of the present invention may vary. Such variation comprises addition, deletion, or non-conservative or conservative substitution of nucleotides.

The nucleic acid is isolated and inserted into a replicable vector to further perform cloning (DNA amplification) or expression. Based on this, in another aspect, the present invention is directed to a vector containing the nucleic acid.

The DNA coding the first and/or second arm of the binding protein is easily separated or synthesized using conventional procedures through the DNA (for example, using an oligonucleotide probe capable of specifically binding to DNA). Vector components generally comprise, but are not limited to, one or more of the following components: signal sequences, replication origins, one or more marker genes, enhancer elements, promoters, and transcription termination sequences.

As used herein, the term "vector" refers to a means for expressing target genes in host cells, and comprises plasmid vectors, cosmid vectors, and viral vectors such as bacteriophage vectors, adenovirus vectors, retroviral vectors, and adeno-associated viral vectors. The nucleic acid encoding the antibody in the vector is operably linked to a promoter.

The term "operably linked" means functional linkage between a nucleic acid expression regulation sequence (e.g., an array of the binding site of the promoter, signal sequence, or transcription regulator) and another nucleic acid sequence, and enables the regulation sequence to regulate transcription and/or translation of the other nucleic acid sequence.

When a prokaryotic cell is used as a host, it generally comprises a potent promoter capable of conducting transcription (such as a tac promoter, lac promoter, lacUV5 promoter, lpp promoter, pLA promoter, pRX promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it comprises a promoter derived from the genome of a mammalian cell (e.g., a metallothionein promoter, a β-actin promoter, a human hemoglobin promoter or a human muscle creatine promoter), or a promoter derived from a mammalian virus (e.g., an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter), and generally has a polyadenylation sequence as a transcription termination sequence.

Optionally, the vector may be fused with another sequence in order to facilitate purification of the antibody expressed thereby. The sequence to be fused therewith may comprise, for example, glutathione S-transferase (Pharmacia, USA), maltose-binding protein (NEB, USA), FLAG (IBI, USA), 6x His (hexahistidine; Quiagen, USA) and the like.

The vector comprises antibiotic resistance genes commonly used in the art as selectable markers, and examples thereof comprise genes conferring resistance to ampicillin, gentamycin, carbenicillin, chloramphenicol, streptomycin, kanamycin, geneticin, neomycin, and tetracycline.

In another aspect, the present invention is directed to a cell transformed with the above-mentioned vector. The cell used to produce the antibody of the present invention may be a prokaryote, yeast, or higher eukaryotic cell, but is not limited thereto.

Prokaryotic host cells such as Escherichia coli, strains of the genus Bacillus, such as Bacillus subtilis and Bacillus thuringiensis, Streptomyces spp., Pseudomonas spp. (for example, Pseudomonas putida), Proteus mirabilis and Staphylococcus spp. (for example, Staphylococcus carnosus) may be used, but is not limited thereto.

Examples of animal cells comprise, but are not limited to, COS-7, BHK, CHO, CHOK1, DXB-11, DG-44, CHO/- DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080, but is not limited thereto.

In another aspect, the present invention is directed to a fusion polypeptide in which an antigen is bound to the polypeptide.

The antigen may be a cancer or tumor antigen for activating an immune response against cancer or tumor. The antigen is a cancer or tumor antigen that may induce cancer- or tumor-specific immune cells, such as cytotoxic T cells, in cell therapy. The cancer or tumor antigen may be, for example, a self or non-self-antigen. For example, cancer or tumor self-antigens derived from the genes of patients may comprise hTERT (GenBank: BAC11010.1), WT1 (GenBank: AAO61088.1), NY-ESO1 (GenBank: CAA05908.1), MAGE-A3 (NCBI Reference Sequence: NP_005353.1), cancer-specific mutant antigens comprise tumor-suppressing or inducing genes such as neoantigens, mutated P53 and RAS, and exogenous cancer or tumor antigens may comprise cancer- or tumor-causing viral antigens such as CMV, EBV and HPV, but is not limited thereto.

hTERT, a self-cancer or tumor antigen, is an enzyme that synthesizes telomeric DNA at the ends of chromosomes, which cancer cells excessively active to evade telomere-dependent cell death, and which is known as a target antigen for various solid cancers comprising lung, stomach and pancreatic cancer (Kim NW, et al. Science. 1994;266:2011-2015), and WT1 is a gene related to Wilms tumor that encodes a zinc finger transcription factor, which is a protein involved in cell proliferation and differentiation, apoptosis, and organ development, and is known as a target antigen for cerebrospinal cancer, lung cancer, or the like (Call KM, et al., Cell. 1990. 60:509-520; Nakahara Y, et al., Brain Tumor Pathol. 2004. 21:113-6). In addition, NY-ESO1 is a protein belonging to CTA (cancer testis antigen), which is well known to be expressed mainly in various cancer cells, comprising germ cells, sarcoma, and breast cancer (Gnjatic S, et al., Adv Cancer Res. 2006;95:1-30). MAGE-A3 is a protein belonging to the melanoma-associated antigen family, which is known to be overexpressed in various cancer cells comprising lung cancer, sarcoma, and melanoma, and is considered as a suitable target antigen for cancer immunotherapy (Decoster L, et al., Ann Oncol. 2012 Jun;23(6):1387-93).

In some cases, the antigen may be, for example, A33, ALK, alpha-fetoprotein (AFP), adrenergic receptor beta 3 (ADRB3), alpha-folate receptor, AD034, AKT1, BCMA, beta-human chorionic gonadotropin, B7H3(CD276), BST2, BRAP, CD5, CD13, CD19, CD20, CD22, CD24, CD30, CD33, CD38, CD40, CD44v6, CD52, CD72, CD79a, CD79b, CD89, CD97, CD123, CD138, CD160, CD171, CD179a, carbonic anhydrase IX (CAIX), CA-125, carcinoembryonic antigen (CEA), CCR4, C-type lectin-like molecule-1 (CLL-1 or CLECL1), claudin 6 (CLDN6), CXORF61, CAGE, CDX2, CLP, CT-7, CT8/HOM-TES-85, cTAGE-1, ERBB2, epidermal growth factor receptor (EGFR), EGFR variant III (EGFRvIII), epithelial cell adhesion molecule (EPCAM), elongation factor 2 mutated (ELF2M), ephrin type-A receptor 2 (EphA2), EMR2, Fms-like tyrosine kinase 3 (FLT3), FCRL5, Fibulin-1, G250, GD2, glycoprotein 36 (gp36), glycoprotein 100 (gp100), glucocorticoid-induced tumor necrosis factor receptor (GITR), GPRC5D, GloboH, G protein-coupled receptor 20 (GPR20), GPC3, hsp70-2, high molecular weight-melanoma-associated antigen (HMWMAA), hepatitis A virus cell receptor 1 (HAVCR1), HAGE, HCA587/MAGE-C2, hCAP-G, HCE661, HER2/neu, HLA-Cw, HOM-HD-21/Galectin9, HOM-MEEL-40/SSX2, HOM-RCC-3.1.3/CAXII, HOXA7, HOXB6, Hu, HUB 1, insulin growth factor (IGF 1)-IL, IGF-II, IGF receptors, interleukin-13 receptor subunit alpha-2 (IL-13Ra2 or CD213A2), interleukin 11 receptor alpha (IL-11Ra), IGLL1, KIT(CD117), KM-HN-3, KM-KN-1, KOC1, KOC2, KOC3, LAGA-1a, LAGE-1, LAIR1, LILRA2, LY75, Lewis Y antigen, MUC1, MN-CA IX, M-CSF, MAGE-1, MAGE-4a, mesothelin, MAGE-A1, MAD-CT-1, MAD-CT-2, MART1, MPPl 1, MSLN, neural cell adhesion molecule (NCAM), NY-ESO-1, NY-ESO-5, Nkp30, NKG2D, mammary differentiation antigen (NY-BR-1), NY-BR-62, NY-BR-85, NY-CO-37, NY-CO-38, NNP-1, NY-LU-12, NY-REN-10, NY-REN-19/LKB/STK1 1, NY-REN-21, NY-REN-26/BCR, NY-REN-3/NY-CO-38, NY-REN-33/SNC6, NY-REN-43, NY-REN-65, NY-REN-9, NY-SAR-35, o-acetyl-GD2 ganglioside (OAcGD2), OGFr, PSMA, prostatic acid phosphatase (PAP), p53, prostate-carcinoma tumor antigen-1 (PCTA-1), prostate stem cell antigen (PSCA), serine protease 21 (testisin or PRSS21), platelet-derived growth factor receptor beta (PDGFR-beta), PLAC1, pannexin 3(PANX3), PLU-1, ROR-1, RAGE-1, RU1, RU2, Rab38, RBPJkappa, RHAMM, stage-specific embryonic antigen-4 (SSEA-4), SCP1, SSX3, SSX4, SSX5, Tyrp-1, TAG72, thyroglobulin, 5T4, tumor-associated glycoprotein 72 (TAG72), tyrosinase, transglutaminase 5 (TGS5), TEM1, TEM7R, thyroid-stimulating hormone receptor (TSHR), Tie 2, TRP-2, TOP2A, TOP2B, uroplakin 2 (UPK2), vimentin, or vascular endothelial growth factor receptor 2 (VEGFR2), but is not limited thereto.

In addition, the antigen is a pathogen (bacteria, virus, etc.), chemical compound, pollen, cancer cell, shrimp, or the like, or some peptide or protein thereof, more preferably a cancer antigen peptide, or any substance causing an immune response in the body, but is not limited thereto. The antigen may preferably be a protein, recombinant protein, glycoprotein, gene, peptide, polysaccharide, lipopolysaccharide, polynucleotide, cell, cell lysate, bacteria, virus, or the like, and more preferably, a cancer antigen peptide. The protein may be an antibody, antibody fragment, structural protein, regulatory protein, transcription factor, toxin protein, hormone, hormone analog, enzyme, enzyme fragment, transport protein, receptor, receptor fragment, biodefense inducer, storage protein, movement protein, exploitive protein, reporter protein or the like. Any substance may be used without limitation as long as it can act as an antigen in the living body and induce an immune response.

The polypeptide and the antigen may be linked through a covalent or non-covalent bond to form a complex and the complex may be incorporated in the immune cells to sensitize the immune cells with the antigen.

In another aspect, the present invention is directed to a nucleic acid encoding the fusion polypeptide according to the present invention. The fusion polypeptide in which the antigen is bound to the polypeptide may be delivered through a vector.

In another aspect, the present invention is directed to an immune cell containing the fusion polypeptide and the nucleic acid. The immune cell may be sensitized with the antigen of the fusion polypeptide.

Sensitization means that an antigen is delivered to immune cells and is loaded onto the surface of cells. The antigen may be prepared in the form of a recombinant antigen with polypeptide to sensitize immune cells. Immune cells may be sensitized by treatment with a polypeptide and an antigen bound with the polypeptide for 24 hours or less. Sensitization time may be controlled depending on the type of antigen and the type of immune cells.

The antigen according to the present invention is an antigen that can bind to the polypeptide according to the present invention and comprises inactivated tumor cells, tumor cell-related genes produced by genetic recombination, peptides, or proteins. In order to obtain an antigen by genetic recombination, a known nucleotide sequence encoding the antigen, a full length of the known sequence or a portion of the full length may be used. The nucleotide sequence encoding the antigen may be cloned into a vector to express the desired antigen.

The antigen may bind to the N-terminus or C-terminus of the polypeptide. The covalent binding may be performed using methods known in the art depending on the type of antigen and may be obtained, for example, by cloning and intracellularly expressing genes encoding polypeptides.

In some cases, linkers that do not interfere with the activity of the polypeptide, for example, N-succinimidyl iodoacetate, N-maleimidobutyryl oxysuccinamide ester, 1,5-difluoro-2,4-dinitrobenzene, bis(diazo)benzidine, 3,3-dithio-bis-(sulfosuccinimidyl-propionate), ethylene glycol bis(sulfosuccinimidyl succinate), dicyclohexyl carbodiimide and the like may be used, but are not limited thereto. On the other hand, if the antigen is active only when decomposed from the polypeptide, a linker that can be cleaved *in vivo* is used. For example, binders with carboxylic acid ester and/or disulfide bonds may be used.

In one embodiment, the immune cells may be antigen-presenting cells (APC) that present on the surface thereof the antigen delivered by the polypeptide according to the present invention. For example, the immune cells may be dendritic cells, monocytes, Wrangel Hans cells, macrophages, T cells, or B cells, but are not limited thereto.

In another aspect, the present invention is directed to an immunotherapeutic agent containing the immune cells. The immunotherapeutic agent according to the present invention may increase an immune response or selectively elevate a part of the immune response that is desirable for the treatment or prevention of a specific disease, infection, or disorder.

Based on this, in another aspect, the present invention is directed to an anti-tumor or anti-cancer vaccine containing the immune cells. The vaccine according to the present invention can improve the immunogenicity of subjects, thereby preventing and/or inhibiting the proliferation and/or metastasis of tumors in the subject.

The vaccine may comprise both immunization performed by single administration and immunization performed by continuous administration.

In another aspect, the present invention is directed to a composition for treating tumors or cancer.

The content and administration method of the active ingredients contained in the composition of the present invention may be determined by those skilled in the art in consideration of the severity of the symptoms and disease of the patient. In addition, the composition may be provided in unit-dose or multi-dose containers, such as sealed ampoules and bottles.

The composition of the present invention may be administered orally or parenterally. The route of administration of the composition according to the present invention comprises, but is not limited to, for example, bronchial, oral, intravenous, intramuscular, intraarterial, intramedullary, intrathecal, intracardiac, transdermal, subcutaneous, intraperitoneal, intestinal, sublingual or topical administration. The dosage of the composition according to the present invention may greatly vary depending on the weight, age, gender, health conditions, and diet of the patient, administration time, method, excretion rate, or severity of the disease, and is easily determined by those skilled in the art. In addition, the composition of the present invention may be prepared into a suitable formulation for clinical administration using known methods.

The pharmaceutical composition may contain a pharmaceutically acceptable carrier. Examples of the pharmaceutically acceptable carrier comprise, but are not limited to, those commonly used for formulation of drugs, comprising lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil and the like. In addition to the ingredients, the pharmaceutical composition according to the present invention may further contain a lubricant, a wetting agent, an emulsifier, a suspending agent, a preservative, or the like.

The appropriate dosage of the pharmaceutical composition of the present invention may be prescribed in various ways depending on factors such as formulation method, administration method, the age, the weight, the gender of the patient, administration time, and administration route. However, severe toxicity (grade of 3 or more) depending on the dosage has not been reported and greatly depends on the preparation method and yield. Meanwhile, the intradermal or subcutaneous dosage of the pharmaceutical composition of the present invention is preferably 0.1×10⁷ to 10×10⁷ cells per administration.

The pharmaceutical composition of the present invention is formulated into a unit dosage form using a pharmaceutically acceptable excipient in accordance with a method that can be easily performed by those skilled in the art. At this time, the formulation may be a suspension in a cell freezing solution or a buffer solution, and may further contain a stabilizer. The immune cell according to an embodiment of the present invention may be frozen after antigen sensitization and thawed as needed.

The dosage of the active ingredient may be appropriately selected depending on various factors such as the route of administration, the age, gender, weight, and severity of the patient, and the composition may be administered in combination with a known compound having effects of preventing, ameliorating, or treating tumor or cancer symptoms.

Hereinafter, the present invention will be described in more detail with reference to examples. However, it will be obvious to those skilled in the art that these examples are provided only for illustration of the present invention and should not be construed as limiting the scope of the present invention.

### Example 1. Research of binding affinity using peptides

### 1.1 Peptide synthesis

Five types of superantigen peptides that bind to MHC class II were selected and synthesized by Anijan Co., Ltd., a peptide manufacturing company.

| Name | Sequence |
|---|---|
| CTP | FITC-linker-YGRRARRRRRR-biotin |
| CTP-SEB-I | FITC-linker-YGRRARRRRRRKSIDQFLYFDLY-biotin |
| CTP-SEB-2 | FITC-linker-YGRRARRRRRRIYSIKDTKLGN-biotin |
| CFP-SEB-3 | FITC-linker-YGRRARRRRRRYYQCYFSKKTNDISHQTDKR KTCM-biotin |
| CTP- SEB-4 | FITC-linker-YGRRARRRRRRYNKKKATVQELD-biotin |
| CTP-SEH | FITC-linker-YGRRARRRRRRDTYCRHNYGV-biotin |

### 1.1.1 ELISA test

To determine the binding ability of the synthesized peptide, MHC class ± protein was coated on a 96-well plate, was treated with 50 nM peptide, and then incubated for 24 hours. The protein was washed 3 times with PBS, incubated with HRP-antibody (1:500) for 2 hours, and washed 5 times with PBS, and a TMB solution was added thereto. The result was incubated for 10 to 30 minutes and color development was stopped with a stopping solution (2MH₂SO₄). After stopping, absorbance was measured at 450 nm to select peptides with excellent binding ability. As a result, the peptide sequence corresponding to SEB2, among the five synthesized peptides, exhibited the highest binding affinity, followed by SEB4, SEB1, and SEB3 in that order.

### 1.1.2 Differentiation of monocyte-derived dendritic cells using PBMC

The PBMC vial was thawed and monocytes were attached to the T150 plate for 30 minutes using 1% AB RPMI medium. Then, unattached cells were removed and cultured in maturation medium conditioned with 30 ng/ml of hGM-CSF and 20 ng/ml of hIL-4 for 3 days.

### 1.1.3 Measurement of peptide cell permeability

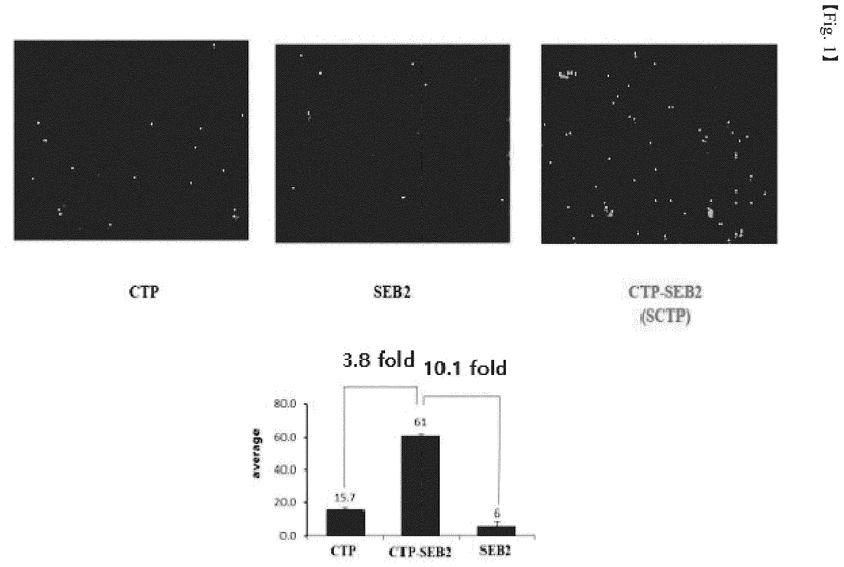

MoDCs were seeded on a cover slip at a confluency of 30 to 40% and then stored at a cell density of 1×10⁴ cells/well in a 6-well plate. The cells were treated with a predetermined amount of each peptide, washed with PBS, and fixed with 4% paraformaldehyde at room temperature for 15 minutes. Then, the cells were blocked using 3% BSA, followed by staining using FITC and DAPI to mark the peptides that had penetrated the cells, and analysis using a fluorescence microscope. As a result, the CTP-SEB2 sequence peptide bound with SEB2, which has a high binding affinity to APC, penetrated MoDCs much more, compared to the CTP sequence peptide of a conventional original patent, whereas the group treated only with the SEB2 sequence peptide did not penetrate the cells. The results are shown in FIG. 1.

### 1.1.4 Measurement of cell permeability using flow cytometry

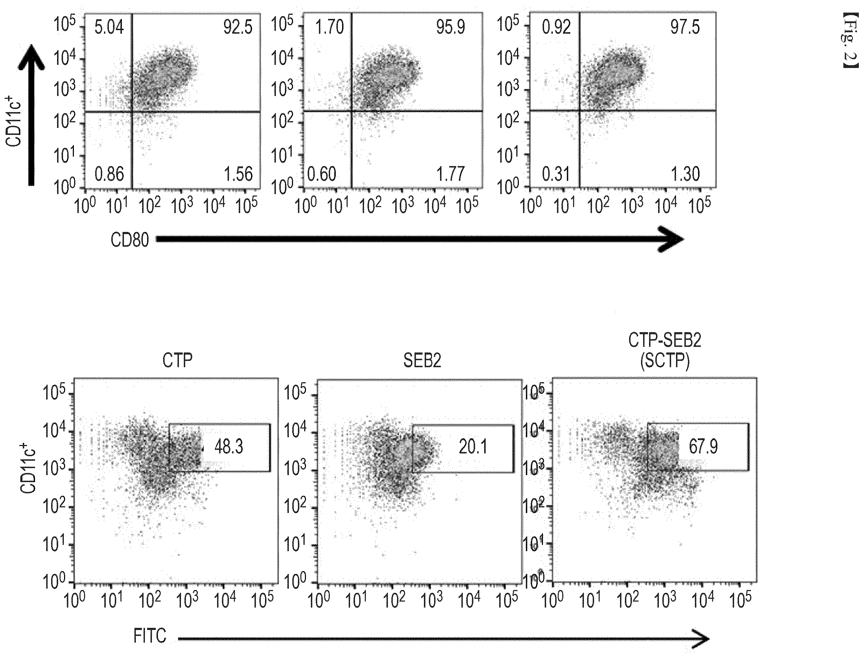

Monocytes were isolated from human peripheral blood and cultured to obtain dendritic cells. Each group was treated with a predetermined concentration of the peptide using the differentiated dendritic cells, incubated for 10 minutes, and then washed with PBS. To analyze the phenotype of dendritic cells, the dendritic cells were stained with anti-mouse CD80, anti-mouse CD86, anti-mouse CD11c, and peptide-FITC and then the phenotypes thereof were analyzed using FACS Canto II. First, after determination of the phenotypes of the produced MoDCs, analysis was conducted to determine whether or not the produced DCs functioned as DCs. The result showed that more than 90% of all the DCs exhibited phenotypes. Then, to analyze peptide permeability in DCs, a double population of CD11c+ and CD80+ was obtained and peptide-FITC within the population was analyzed. The result of analysis showed that, like the microscopic results, the CTP-SEB2 peptide group permeated a large amount of peptide compared to the conventional CTP peptide group, whereas the SEB2-only peptide had almost no permeability. The results are shown in FIG. 2.

### Example 2. Production of peptide fusion protein fused to CTP

### 2.1 Construction of fusion protein vector

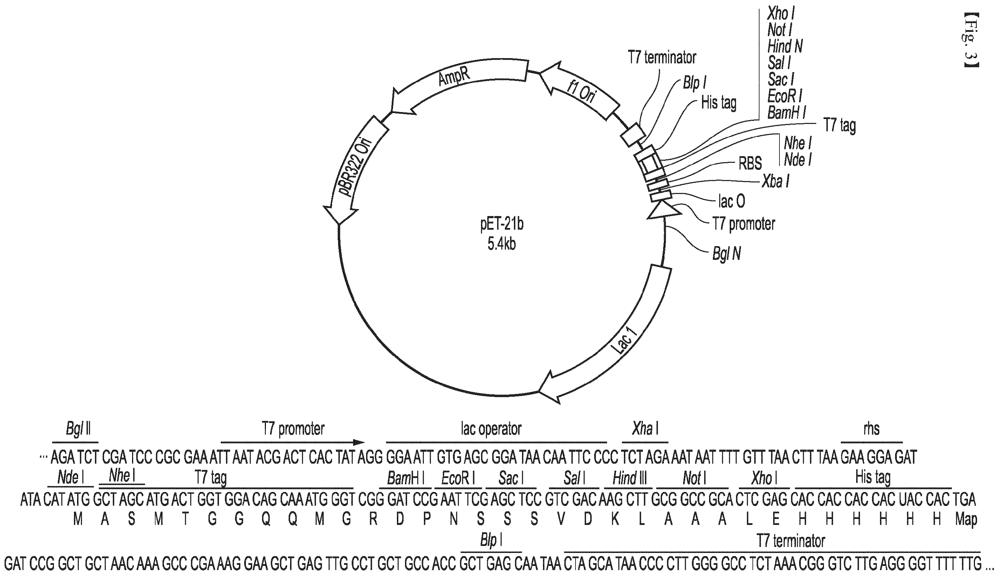

The SEB2 sequence peptide found to have the best binding ability based on the result of permeability test was selected and a recombinant protein vector was produced (Genscript, Piscataway, NJ) as shown in FIG. 3.

### 2.2 Production of recombinant fusion protein

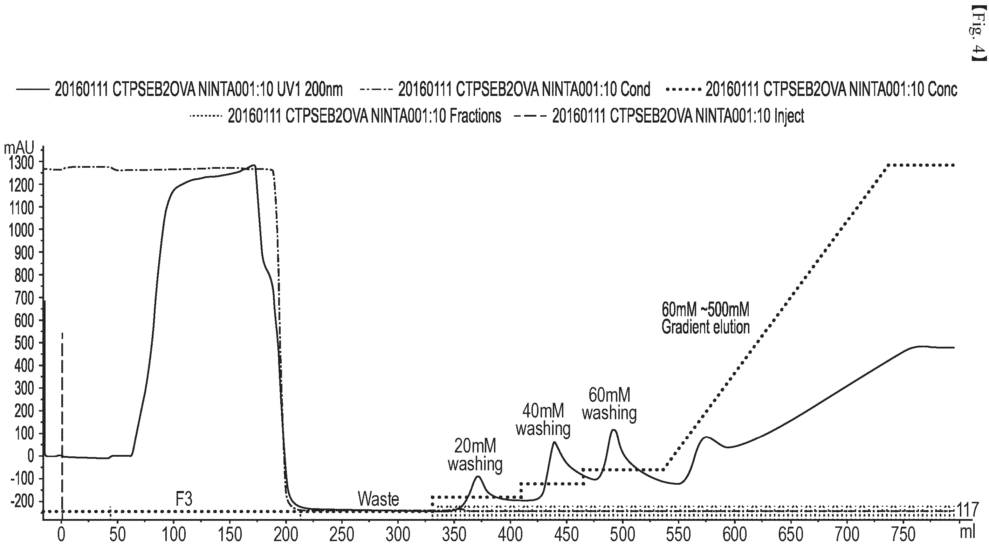

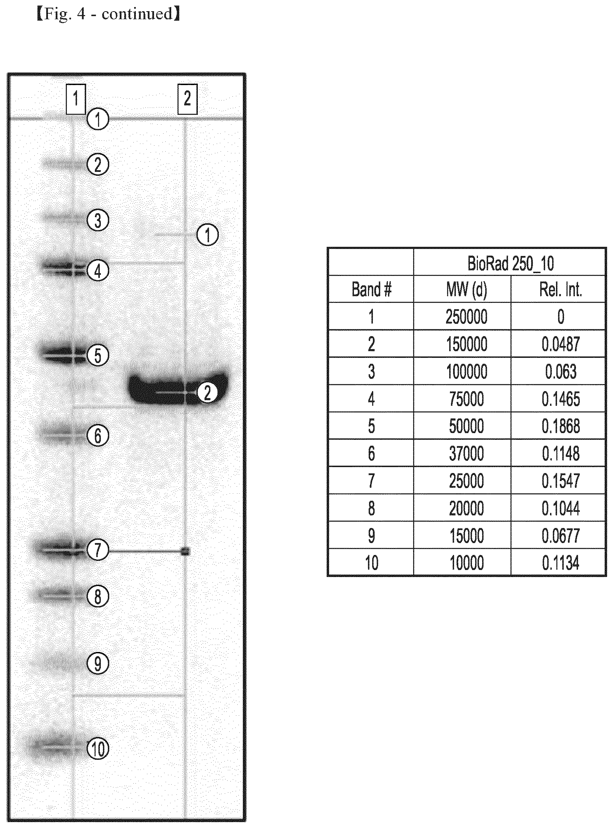

The recombinant gene was cultured using *E. coli,* produced in IB state, solubilized and then separated and purified using a Ni-NTA resin. Specifically, first, the gene was seed-cultured at 37°C overnight, and then incubated at 37°C in a LB broth miller medium (Novagen, yeast extract 5 g, peptone from casein 10 g, sodium chloride 10 g) supplemented with 50 µg/ml of ampicillin. When the absorbance at OD₆₀₀ nm reached about 0.8 to about 1.0, IPTG was added to adjust a final concentration to 0.5 mM to induce protein expression, followed by culture with shaking at 180 rpm for 8 hours. Bacterial cells that had grown to an absorbance of 1.5 to 2.0 at OD₆₀₀ nm were precipitated by centrifugation at 10,000 rpm at 4°C for 20 minutes, followed by stirring until the cells were completely suspended in 50 ml of disintegration buffer (300 mM NaCl, 50 mM Tris (pH 8.0), 0.5% Triton X-100) per 2 g (1L culture volume) of precipitated cells. PMSF was added to a final concentration of 1 mM, and the cells were lysed using an ultrasonicator. The lysed cells were centrifuged at 12,000 rpm at 4°C for 30 minutes to separate the suspension from the total lysate. Then, only the suspension was collected, filtered through a cellulose filtration membrane with a pore size of 0.45 um, and then loaded on a nickel affinity chromatography column that was previously equilibrated with an equilibration buffer. The purified protein was stabilized through buffer exchange and then QC was performed. The results are shown in FIG. 4.

### 2.3 Measurement of cell permeability of fusion protein

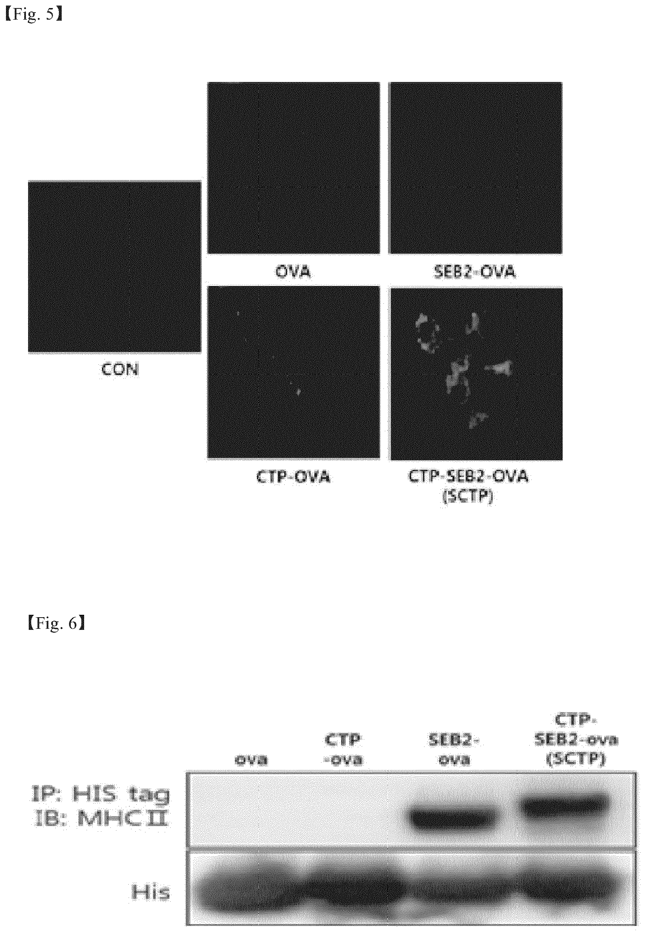

MoDCs were seeded on a cover slip at a confluency of 30 to 40% and then stored at a cell density of 1 × 10⁴ cells/well on a 6-well plate. The cells were treated with a predetermined amount of each peptide, incubated, washed with PBS, and fixed with 4% paraformaldehyde at room temperature for 15 minutes. Then, the cells were blocked using 3% BSA, followed by staining using Cy5.5 and DAPI to mark the proteins permeating the cells, followed by analysis using a fluorescence microscope. The result showed that a large amount of protein of the CTP-SEB2-OVA group, which was a group treated with the recombinant protein, permeated the cells, compared to the untreated group, and that the CTP-SEB2-OVA group had a greater amount of permeation, compared to the group treated with the CTP-OVA protein produced with the conventional CTP sequence. On the other hand, protein permeation was not possible in the OVA and SEB-OVA groups that did not contain CTP. The results are shown in FIG. 5.

### 2.4 Measurement of fusion protein cell permeability using Western blot

The cells were washed with cold PBS and then lysed in lysing buffer containing 50 mM Tris-HCl (pH 7.4), 150 mM NaCl, 1 mM DTT, 30 mM NaF, 10 mM Na₃VO₄, 0.5% NP40 and Pierce (protease inhibitor cocktail). Whole cell lysates were adjusted to the same concentration using Bradford agent (Bio-Rad), and 40 to 120 µg of the lysate was analyzed on an 8-12% acrylamide gel using SDS-PAGE and transferred to a PVDF membrane (Milipore). For immunoblotting, the membrane was blocked with 5% non-fat dry milk prepared in TBS (TBST) containing 0.5% Tween20 at room temperature for one hour. The membrane was washed four times with TBST, treated with a primary antibody diluted in 4% non-fat dry milk at the optimal concentration, and incubated overnight at 4°C. After washing four times with TBST, the cells were incubated with HRP-conjugated secondary antibody for 45 minutes. The bound antibody was detected using a chemiluminescent HRP substrate (Millipore, USA) and Chemi DOC (Bio-RAD, USA).

### 2.5 Analysis of direct binding affinity of fusion proteins

A gene expressing HIS-tag was produced using the selected peptide, a protein was produced and whether or not the protein directly interacted with MHC class II was determined by immunoprecipitation of His.

Specifically, 4×10⁷ cells were collected and washed twice with PBS, and cells suspended in a protease inhibitor cocktail [2 mM AEBSF, 1 mM EDTA, 130 µM Bestatin, 1 µM leupeptin, 14 µM E-64, 0.3 µM aprotinin] and NP-40 lysis buffer (20 mM Tris, pH 7.4, 150 mM NaCl, 1% NP-40, 10% glycerol) containing a dephosphatase inhibitor (Roche) were rotated on a rotator in a 4°C chamber for 30 minutes (C1, 25 rpm). The cells were centrifuged at 13,000 rpm and 4°C for 10 minutes and the supernatant was collected. Then, the residue was centrifuged again at 12,000 rpm and 4°C for 10 minutes to collect only the clean supernatant. The protein was assayed, and the protein used as cell lysate data was sampled separately and stored at -80°C. The protein for immunoprecipitation was fragmented in 500 µg or more/700 to 1,000 µl and the balance was filled to a total of 1 mL with a lysate buffer. The antibody for immunoprecipitation was added at 1 to 2 µg per 100 to 500 µg of protein and was rotated on a rotator in a 4°C chamber for 2 hours or longer or overnight (C1, 10 rpm). Then, about 30 to 50 µl of protein A/G beads (Pierce) was rotated on a rotator in a 4°C chamber for 1 to 2 hours. The result was washed three times with NP-40 lysate buffer at 1,200 rpm, 3 min, and 4°C. After washing, the remaining buffer was completely removed and boiled in 20 µl of 5X sample buffer. The result was cooled in ice, the mixture was centrifuged for 2 minutes, and only the supernatant was collected and separated using 7.5 to 12% SDS-PAGE. The gel was blotted again with a PVDF membrane, a primary antibody was attached to the membrane to which the protein had been transferred, a secondary antibody IgG was attached thereto and the result was observed using an ECL detection kit.

The analysis results are shown in FIG. 6. The result showed that, when protein A/G beads bound to the fusion protein were subjected to Western blot using the target protein, the protein fused with the peptide was detected along with the target protein, whereas the protein having no peptide was not detected.

### 2.6 Mouse dendritic cell isolation

Bone marrow stem cells were differentiated into dendritic cells (DCs) and then cultured. More specifically, bone marrow-derived dendritic cells (BMDC) were isolated from the femur and tibia of 6-8 week old female C57BL/6 mice, and then treated with ACK lysis buffer (Lonza) to remove red blood cells. The cells were washed and cultured with a culture medium (RPMI 1640 containing 10% FBS and penicillin/streptomycin) containing 10 ng/ml of mGM-CSF (Creagene Inc.). After 2 days, the supernatant of the cultured cells was removed and replaced with 2 ml of a fresh culture medium containing mGM-CSF. On the 4^{th} day of culture, 1 ml of a fresh culture medium containing mGM-CSF was added. On the 6^{th} day of culture, non-adherent cells were collected and used as immature dendritic cells (immature DCs, imDC). The mature dendritic cells (mature DCs, mDCs) were prepared by culturing immature dendritic cells (immature DC) in the presence of LPS (100-200 ng/ml) for 24 hours.

### 2.7 Measurement of cell permeability using flow cytometry

Monocytes were isolated from mouse bones and cultured to obtain BMDCs. Using the differentiated dendritic cells, each group was treated with a predetermined concentration of peptide, cultured for 1 hour, and then was washed with PBS. To analyze the phenotypes of dendritic cells, the dendritic cells were stained with anti-mouse CD80, anti-mouse CD86, anti-mouse CD11c, and peptide-FITC and then the phenotypes thereof were analyzed using FACS Canto II. The permeability of peptide in CD11c+ was measured by analysis using peptide-FITC.

### 2.8 Analysis of protein residue in culture medium using TCA precipitation

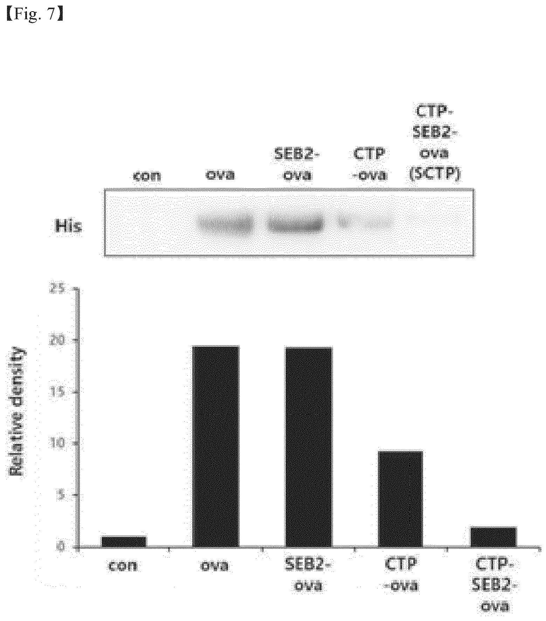

A 100% TCA solution was added to a culture sample at a volume ratio of 1/10, vortexed vigorously, allowed to stand in ice for 30 minutes, and then centrifuged at 4°C and 14,000 rpm for 10 minutes. Then, the supernatant was discarded and 500 µl of cold acetone was added to the precipitate, followed by centrifugation at 4°C and 14,000 rpm for 10 minutes to remove TCA. Then, the precipitate was dried and resuspended using a buffer solution, and Western blotting was performed. As shown in FIG. 7, as a result of measurement of the protein residue present in the medium, most of a peptide-bound CTP-SEB2-OVA protein penetrated the cells and the amount of protein present in the medium was not large, whereas the amount of CTP-OVA conventional sequence protein penetrating the cells was slightly low. However, all proteins to which CTP was not bound were present in the medium.

### 2.9 Test of comparison in PTD-based peptide cell membrane permeability

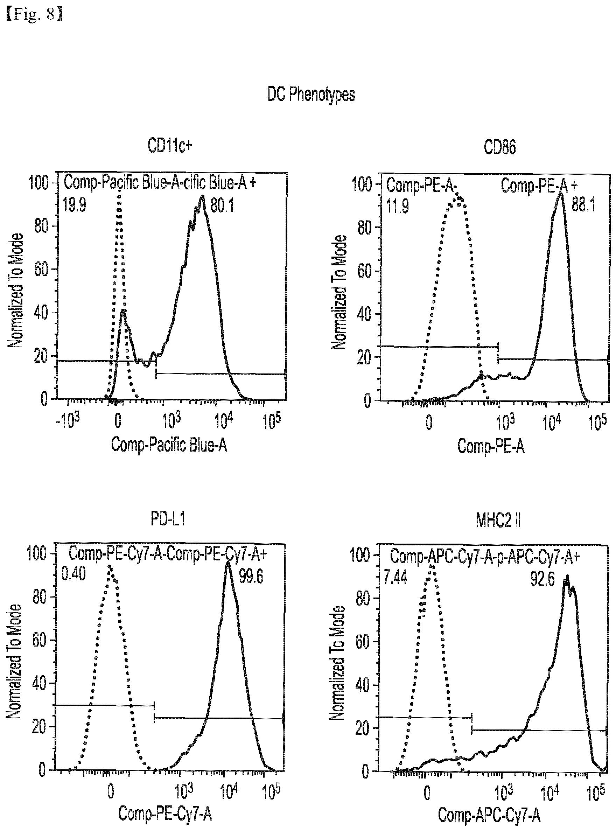

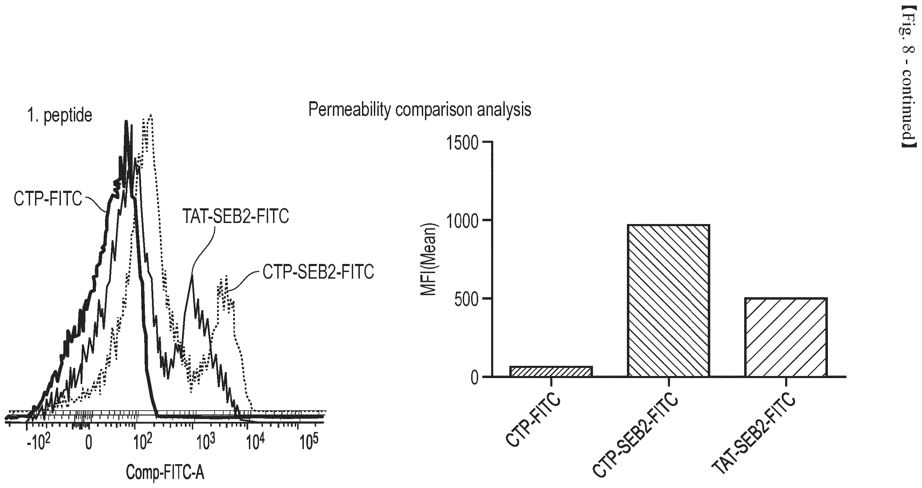

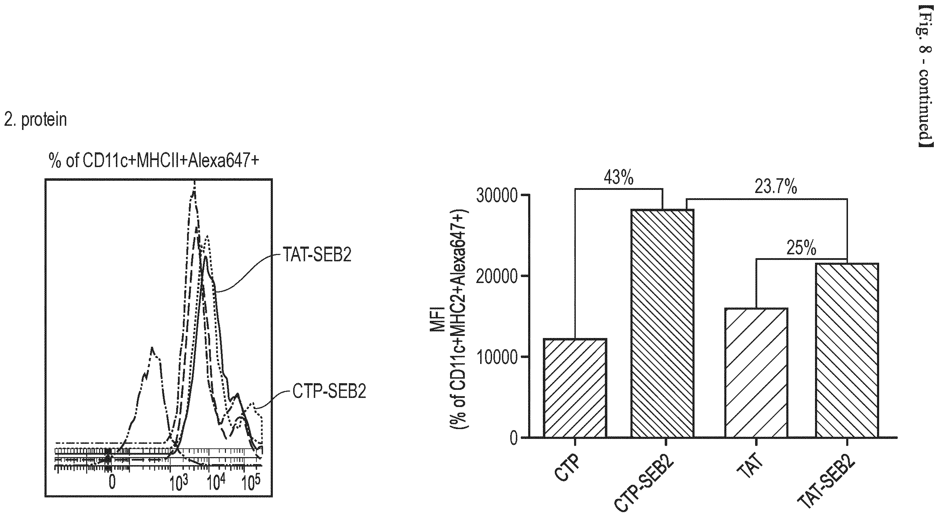

The permeability was compared between the peptide sequence currently developed by the present inventors and the previously known PTD-based transduction peptide sequence to demonstrate that the sequence currently developed by the present inventors has superior permeability. BMDCs were seeded at 2×10⁵/ml on a 12-well plate and cultured at 37°C for 1 day. The cells were treated with 1 µM CTP-FITC, TAT-SEB2-FITC, and CTP-SEB2-FITC peptides (Anygen Co., Ltd.) attached to the cell surface for 4 hours, and the degree of fluorescent labeling of cells was determined by FACS. First, the DC phenotypes in mouse BMDCs were analyzed. The result showed that BMDCs were produced well with a yield of over 90%. The DCs were treated with peptides. The results showed that the permeability effect is greater when SEB2 is combined with the CTP sequence of the present inventors than when the SEB2 sequence is combined with the previously known PTD peptide. This was also the same as the result of the fusion protein (FIG. 8).

### 2.10 Antigen-specific T cell proliferation analysis

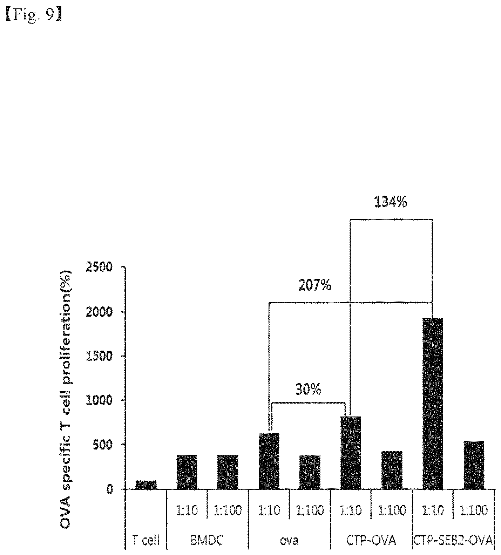

T cell proliferation was analyzed through co-culture with DCs using spleens from TCR (OT-I) transgenic mice specific for OVA (ovalbumin) antigens. MLR measurement to determine the functions of cultured cells was performed by culturing splenocytes (R, OT-1 specific T cells: 1×10⁵ cells/well) and DCs (S, stimulator) treated with an OVA antigen at a ratio (R:S) of 1:10 to 1:100 on a 96-well flat-bottomed microtiter plate for 96 hours and then measuring the degree of proliferation of responder cells. As shown in FIG. 9, the group containing DCs sensitized with the peptide-fused antigen exhibited the highest OVA-specific T cell response, whereas the CTP antigen-sensitized DCs exhibited increased specific T cell response, but exhibited lower reactivity than that of the peptide-fused antigen.

### Example 3. Effect of dendritic cell vaccine on mouse melanoma prevention (Prevention model)

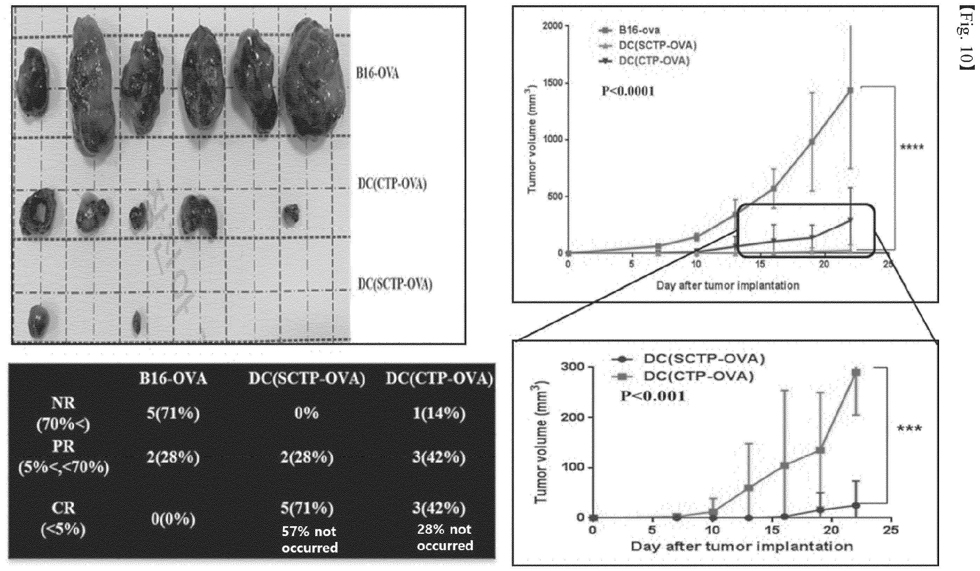

To determine the possibility of melanoma prevention by dendritic cell vaccine, mice were sensitized twice with dendritic cells sensitized with CTP-fusion melanoma recombinant antigen, and then a challenge test was conducted with cancer cell lines expressing melanoma-specific antigens to determine the formation of solid tumors. Mouse dendritic cells used herein were bone marrow cells of the femur and tibia differentiated into dendritic cells. Both ends of the femur and tibia were cut to extract bone marrow cells and the cells were collected in a 50 ml tube. The collected bone marrow cells were suspended in 0.83% ammonium chloride solution to remove red blood cells, and the bone marrow cells were cultured in dendritic cell production medium (RPMI-1640, 10% FBS, 10 ng/ml of mouse recombinant IL-4 and 10 ng/ml of mouse GM-CSF) for 2 days, non-adherent cells were removed, and only cells attached to the bottom of the container were collected. The medium was replaced with fresh medium every 2-3 days to prevent depletion of cytokines. On the 6^{th} day of culture, immature dendritic cells were collected and treated with the recombinant antigens CTP-OVA and SCTP-OVA. Immature dendritic cells were sensitized by treatment with 10 pg/ml of each antigen protein (Korean Patent No. 10-0647847) for 20 hours. At 24 hours, cytokines required for dendritic cell maturation (100 ug/ml of IFN-γ and 100 ug/ml of TNF-α) were added to induce maturation of dendritic cells. Anticancer immunity was induced by subcutaneously injecting 1×10⁶ antigen-sensitized dendritic cells into mice. Sensitized Dendritic cell was administered twice at 1-week intervals. 1 week after the secondary dendritic cell inoculation, B16-OVA melanoma cells were SC (subcutaneously) injected at 1×10⁶ cells/mouse. The size of the cancer (width × height) was measured every 2 days. The result of the measurement showed that the experimental group inoculated with dendritic cells sensitized with CTP-OVA or SCTP-OVA antigen exhibited the antitumor effect, as shown in FIG. 10.

### Example 4. Analysis of immune cell permeability using PBMC

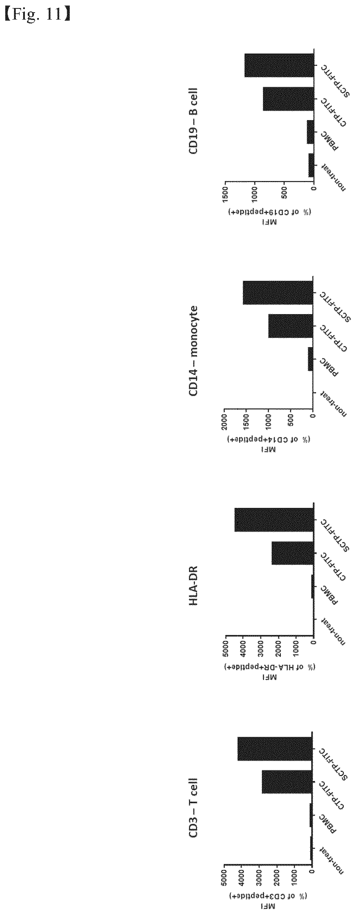

The permeability of the fusion peptide to be developed in various immune cells (monocytes, T cells, B cells, dendritic cells, NK cells, and the like) present in PBMCs was measured. For this purpose, frozen PBMCs were thawed, treated with a concentration of 1 µM of each of the conventional CTP peptide and the fused CTP-SEB peptide, and then analyzed by FACS. Each immune cell was identified using representative markers of immune cells such as CD14 (monocytes), CD3 (T cells), CD19 (B cells), and HLA-DR (dendritic cells) and the amounts of permeated peptides were analyzed. The peptide permeability of each immune cell was measured. As shown in FIG. 11, the result showed that the fusion peptide was more permeable than the conventional CTP peptide in monocytes, B cells, and dendritic cells known to express HLA-DR and the fusion peptide was more permeable in T cells. This indicates that the overall fusion peptide in immune cells increased permeability.

### [Industrial applicability]

The polypeptide according to the present invention has an excellent ability to penetrate the cell membrane of immune cells and directly targets immune cells to deliver antigens with excellent efficiency, thereby being effective in proliferating T cells.

Although specific configurations of the present invention have been described in detail, those skilled in the art will appreciate that this detailed description is provided as preferred embodiments for illustrative purposes and should not be construed as limiting the scope of the present invention. Therefore, the substantial scope of the present invention is defined by the accompanying filed claims and equivalents thereto.

### [Sequence Listing Free Text]

An electronic file is attached.

## Claims

1. A polypeptide for delivering an antigen to an immune cell comprising:
a cytoplasmic transduction peptide (CTP); and
a peptide binding to a surface molecule of the immune cell.

2. The polypeptide according to claim 1, wherein the surface molecule of the immune cell is major histocompatibility complex class II (MHC class II).

3. The polypeptide according to claim 1, wherein the immune cell is a dendritic cell, a monocyte, a Wrangel Hans cell, a macrophage, a T cell, or a B cell.

4. The polypeptide according to claim 1, wherein the peptide binding to the surface molecule of the immune cell comprises a sequence selected from the group consisting of SEQ ID NOS: 1 to 4.

5. The polypeptide according to claim 1, wherein the cytoplasmic transduction peptide (CTP) comprises a sequence selected from the group consisting of SEQ ID NOS: 5 to 7.

6. The polypeptide according to claim 1, wherein the peptide binding to the surface molecule of the immune cell is fused with an end of the cytoplasmic transduction peptide.

7. The polypeptide according to claim 1, wherein the antigen is a cancer or tumor antigen for activating an immune response against cancer or a tumor.

8. A nucleic acid encoding the polypeptide according to any one of claims 1 to 7.

9. A fusion polypeptide in which an antigen is bound to the polypeptide according to any one of claims 1 to 7.

10. The fusion polypeptide according to claim 9, wherein the antigen is a cancer or tumor antigen for activating an immune response against cancer or a tumor.

11. A nucleic acid encoding the fusion polypeptide according to claim 9.

12. An immune cell comprising the fusion polypeptide according to claim 9.

13. The immune cell according to claim 12, wherein the immune cell is a dendritic cell, a monocyte, a Wrangel Hans cell, a macrophage, a T cell, or a B cell.

14. An immunotherapeutic agent comprising the immune cell according to claim 12.

15. An anti-tumor or anti-cancer vaccine comprising the immune cell according to claim 12.

16. A composition for treating tumors or cancer comprising the immune cell according to claim 12.

17. An immune cell comprising the nucleic acid according to claim 11.

18. The immune cell according to claim 17, wherein the immune cell is a dendritic cell, a monocyte, a Wrangel Hans cell, a macrophage, a T cell, or a B cell.

19. An immunotherapeutic agent comprising the immune cell according to claim 17.

20. An anti-tumor or anti-cancer vaccine comprising the immune cell according to claim 17.

21. A composition for treating tumors or cancer comprising the immune cell according to claim 17.
